(19) Europäisches Patentamt

European Patent Office

Office européen des brevets

(11) EP 0 978 690 A2

(12) EUROPEAN PATENT APPLICATION

(43) Date of publication:
09.02.2000 Bulletin 2000/06

(51) Int. Cl.7: F24F 3/16

(21) Application number: 99107320.6

(22) Date of filing: 20.04.1999

(84) Designated Contracting States:
**AT BE CH CY DE DK ES FI FR GB GR IE IT LI LU MC NL PT SE**
Designated Extension States:
**AL LT LV MK RO SI**

(30) Priority: 05.08.1998 JP 23495898

(71) Applicant:
**NITTO DENKO CORPORATION**
**Osaka (JP)**

(72) Inventors:
• **Ikebata, Hisashi,**
**Nitto Denko Corporation**
**Ibaraki-shi, Osaka (JP)**
• **Ishizaki, Akira,**
**Nitto Denko Corporation**
**Ibaraki-shi, Osaka (JP)**

(74) Representative:
**Grünecker, Kinkeldey,**
**Stockmair & Schwanhäusser**
**Anwaltssozietät**
**Maximilianstrasse 58**
**80538 München (DE)**

(54) **Air cleaning unit**

(57) An air cleaning unit having a photocatalyst sheet comprising a supporting substrate having thereon a photocatalyst layer comprising a baked polytetrafluoroethylene resin having dispersed therein photocatalyst particles with fine voids formed between the resin and the photocatalyst particles, the photocatalyst sheet being placed in an area receptive of light from an ultraviolet generator.

FIG. 1

## Description

### FIELD OF THE INVENTION

This invention relates to an air cleaning unit comprising an air-permeable photocatalyst sheet, which is useful for the purification of air such as removal or reduction of bad odors such as the odor of tobacco or harmful gases, or microorganisms.

### BACKGROUND OF THE INVENTION

As is well known, when a metal oxide semiconductor such as titanium oxide is irradiated with ultraviolet light, electrons in the valency band are lifted up to the conduction band to make holes, and active species (radicals) are generated from oxygen or moisture in contact with the thus excited surface of the metal oxide semiconductor. The active species oxidatively decompose organic matter, microorganisms, etc. attached to the surface. They also oxidize $NO_x$ or $SO_x$ to the final oxide.

A photocatalyst sheet comprising a supporting substrate having carried thereon fine particles of such a metal oxide semiconductor as a photocatalyst is known as an air cleaning means which is, on being irradiated with ultraviolet light, capable of removing or reducing bad odors, such as the odor of tobacco, harmful gases, such as $NO_x$ and $SO_x$, volatile organic compounds (that are generated in new houses), and microorganisms in a living space or a working environment.

The photocatalyst particles are usually held on the substrate by a binder. The binder for this use is required to be stable against decomposition by the activated photocatalyst particles. From this standpoint, a number of proposals have been disclosed. For example, JP-A-7-171408 teaches using silicone resins or fluororesins, such as a vinyl ether-fluoroolefin copolymer and a vinyl ester-fluoroolefin copolymer, as a binder.

Various air cleaning units comprising the above-described photocatalyst sheet have been proposed (see, e.g., JP-A-3-106420, JP-A-7-251028, JP-A-7-35373, and JP-A-7-284523), but they have low air cleaning efficiency because most of the surface of the photocatalyst particles is covered with the binder resin only to provide a small contact area with air. This has been a bar to reduction of size and cost of the air cleaning unit.

The inventors of the present invention have conducted investigations aiming at improvement in deodorizing performance of a photocatalyst sheet. They have found as a result that a photocatalyst layer prepared by applying a dispersion of polytetrafluoroethylene powder and photocatalyst particles to a substrate followed by baking exhibits markedly excellent deodorizing performance. Microscopic observation of the photocatalyst layer revealed void layers between the individual photocatalyst particles and the surrounding binder resin, which are connected to each other to form a continuous passage. Void formation in the interface between the photocatalyst particles and the polytetrafluoroethylene resin seems attributable to, for one thing, the large difference in thermal shrinkage between polytetrafluoroethylene and titanium oxide and, for another, non-adhesion of polytetrafluoroethylene. It is assumed that a great thermal shrinkage stress develops in the interface on cooling following baking and that the great tensile stress causes interfacial separation due to the interfacial non-adhesion.

According to conventional processes for producing a photocatalyst sheet comprising applying a dispersion of a fluororesin (e.g., a vinyl ether-fluoroolefin copolymer or a vinyl ester-fluoroolefin copolymer), a crosslinking agent (e.g., an isocyanate curing agent), and photocatalyst particles to a substrate and curing the coating layer through crosslinking (see JP-A-7-171408 supra), there is included no such step that would generate a shrinkage stress in the interface between the photocatalyst particles and the resin to form voids.

### SUMMARY OF THE INVENTION

An object of the present invention is to provide an air cleaning unit comprising a photocatalyst sheet in which the photocatalyst sheet has excellent deodorizing performance and is practical for size and cost reduction of the unit.

The present invention provides an air cleaning unit having a photocatalyst sheet comprising a supporting substrate having thereon a photocatalyst layer comprising a baked polytetrafluoroethylene resin having dispersed therein photocatalyst particles with fine voids formed between the resin and the photocatalyst particles, the photocatalyst sheet being placed in an area receptive of light from an ultraviolet generator.

The photocatalyst sheet can be a laminate with a deodorant sheet mainly comprising one or more of activated carbon, zeolite and copper carboxymethylcellulose. The photocatalyst sheet can be an air-permeable sheet comprising a mesh or perforated supporting substrate, which is placed in a curved or folded form in an area receptive of light from an ultraviolet generator. The void volume of the photocatalyst layer is preferably 7% or more.

According to the present invention, air is brought into contact with almost all the surface area of UV-activated photocatalyst particles in the photocatalyst sheet while passing through microvoid passages formed between the photocatalyst particles and the binder resin. Therefore, the oxidative deodorizing performance of the activated photocatalyst particles can be maximized to achieve improved air cleaning efficiency. Further, the photocatalyst particles are kept supported stably during their service life, being enveloped in the binder resin that is hardly decomposable. Therefore, the air cleaning unit of the invention keeps on cleaning air at high efficiency for an extended period of time.

## BRIEF DESCRIPTION OF THE DRAWINGS

Fig. 1 is a cross section of the photocatalyst sheet used in the invention.
Fig. 2A is a plan view of the air-permeable photocatalyst sheet used in the invention, and Fig. 2B is a cross section of Fig. 2A along line B-B.
Fig. 3A illustrates a plane view of an example of the air cleaning unit of the invention, and Fig. 3B is a cross section of Fig. 3A along line B-B.
Fig. 4A shows a plane view of another example of the air cleaning unit according to the invention, and Fig. 4B is a cross section of Fig. 4A along line B-B.
Figs. 5A to 5D and Fig. 6A to 6D show other examples of the air cleaning unit according to the invention.

## PREFERRED EMBODIMENTS OF THE INVENTION

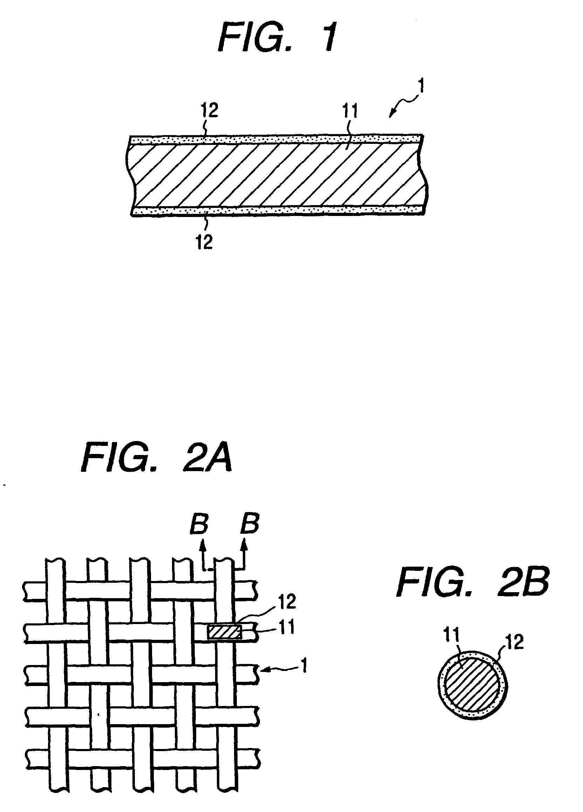

Fig. 1 shows a cross section of a photocatalyst sheet 1 used in the invention. The photocatalyst sheet 1 is composed of a supporting substrate 11 and a pair of photocatalyst layers 12 provided on substrate 11. The photocatalyst layer 12 is a baked layer made up of sintered polytetrafluoroethylene resin having dispersed therein photocatalyst particles. Microvoids are formed between the resin and the photocatalyst particles, and the interstices among sintered polytetrafluoroethylene particles are linked to the layer of the microvoids to form a structure comprising many voids interconnected, which form a continuous passage for air.

The voids formed between the polytetrafluoroethylene resin and the photocatalyst particles have a very small thickness of from several nanometers to several microns. They do not allow water, etc. to pass due to water repellency of polytetrafluoroethylene but allow air to permeate sufficiently.

Fig. 2A is a plan view of the air-permeable photocatalyst sheet used in the invention, and Fig. 2B is a cross section of Fig. 2A along line B-B. In Figs. 2A and 2B numeral 11 is a mesh substrate (e.g., glass cloth of plain weave), and numeral 12 is a photocatalyst layer provided on the substrate 11. As mentioned above, the photocatalyst layer 12 is a baked layer of sintered polytetrafluoroethylene resin having dispersed therein photocatalyst particles with microvoids formed between the resin and the photocatalyst particles. The gaps among sintered polytetrafluoroethylene particles are linked to the layer of the microvoids to provide continuous passages for air.

The photocatalyst sheet can be prepared by coating a supporting substrate with a dispersion comprising polytetrafluoroethylene powder, photocatalyst particles, and a dispersion medium (e.g., water), heating the coating layer to evaporate the dispersion medium, and baking the coating layer at a baking temperature of 330°C or higher to sinter the polytetrafluoroethylene powder. On cooling following the baking, a void layer is formed between the photocatalyst particles and the polytetrafluoroethylene resin due to greater thermal shrinkage of the resin than that of the photocatalyst particles and the non-adhesion of the resin to the photocatalyst particles.

Because the polytetrafluoroethylene has a high melt viscosity ($10^8$ poise or greater) during baking, it retains its particulate shape without flow. Because the sintering is effected with no pressure applied, the voids sufficiently remain among sintered polytetrafluoroethylene particles. As a result, the resulting photocatalyst layer has an air-permeable structure having many voids. The baking is generally effected at a temperature of from 330 to 400°C, preferably from 360 to 380°C and preferably for 2 to 3 minutes.

The polytetrafluoroethylene powder usually has a particle size of 0.1 to 1 μm. The commercially available dispersion of polytetrafluoroethylene powder, which is prepared by emulsion polymerization, may be used.

The photocatalyst particles usually have a particle size of 200 nm or smaller, preferably 50 nm or smaller. The lower limit of the particle size of the photocatalyst particles is generally 5 nm in terms of the primary particle size.

It is preferred for the photocatalyst layer to have a void volume of 7% or more, particularly 10% or more. The void volume x as referred to above is given by equation:

$$x = 1 - [w/(v\rho)]$$

wherein $\rho$ is a true specific gravity of the photocatalyst layer; $v$ is the volume of the photocatalyst layer; and $w$ is the weight of the photocatalyst layer.

The predetermined void volume can be provided by appropriately selecting the photocatalyst particles and conditions of heating and cooling as described above.

Where the void volume is less than 7%, the effect in improving the contact of air with the photocatalyst particles through the structure comprising many voids is lessened. It is preferred, however, that the void volume be not more than 30% from the standpoint of mechanical strength of the photocatalyst layer.

The thickness of the photocatalyst layer is preferably 3 to 30 μm. With a thickness smaller than 3 μm, the so reduced volume of the photocatalyst layer has reduced air cleaning performance. With a thickness exceeding 30 μm, the gas diffusion efficiency is reduced, whereby it is necessary to provide a thickness more than the thickness expected to be necessary.

The content of the photocatalyst particles in the dispersion is preferably 5 to 60% by weight, more preferably 30 to 50% by weight, based on the weight of the photocatalyst layer (i.e., total weight of solid components). If the dispersion has too high a photocatalyst particles content, the binding strength among the photocatalyst particles by the polytetrafluoroethylene resin would be insufficient.

The photocatalyst which can be used in the invention includes titanium oxide, strontium titanate, tungsten oxide, zinc oxide, tin oxide, and cadmium sulfide. Anatase type titanium oxide having the most excellent photocatalyst activity is particularly preferred. The alkali metal ions can be carried on the photocatalyst particles to enhance the activity of the photocatalyst particles.

The substrate having heat resistance against deformation on baking can be used in the present invention. Examples of useful substrates include metal foil, such as aluminum foil and stainless steel foil; an inorganic plate, such as a ceramic plate or a glass plate; a heat-resistant plastic film, such as polyimide film or a polytetrafluoroethylene film; woven fabric of heat-resistant plastic (e.g., polytetrafluoroethylene)-impregnated glass fibers or polyamide fibers; a felt-like product of glass fibers, ceramic fibers, metal fibers or carbon fibers or a mixture thereof; and a net-like product of glass fibers, ceramic fibers, metal fibers or carbon fibers or a mixture thereof.

The mesh or perforated substrate to be used for the air-permeable photocatalyst sheet is not particularly limited as far as such requirements as flexibility, heat resistance and strength for withstanding the baking are fulfilled. Examples of useful substrates are metal net, e.g., aluminum net, a punched sheet of a heat-resistant plastic, e.g., polyimide or polytetrafluoroethylene, and woven fabric of heat-resistant organic or inorganic fiber, e.g., glass fiber or polyamide fiber.

The dispersion is applied to the substrate by roll coating, dip coating, spray coating, brush coating, casting, or a like coating method. The concentration of the solid components in the dispersion usually ranges from 40 to 60% by weight while varying according to the coating method and the like. If desired, the dispersion can contain additives for increasing the void volume of the baked layer, additives for enhancing the strength, and gas adsorbents that withstand the baking temperature, preferably in an amount of 20% by weight or less.

The above-described photocatalyst sheet can be used alone or as a laminate with a deodorant sheet mainly comprising one or more of activated carbon, zeolite and copper carboxymethylcellulose. It is also possible to incorporate one or more of activated carbon particles, zeolite particles and silica gel particles, along with the photocatalyst particles, into the photocatalyst sheet.

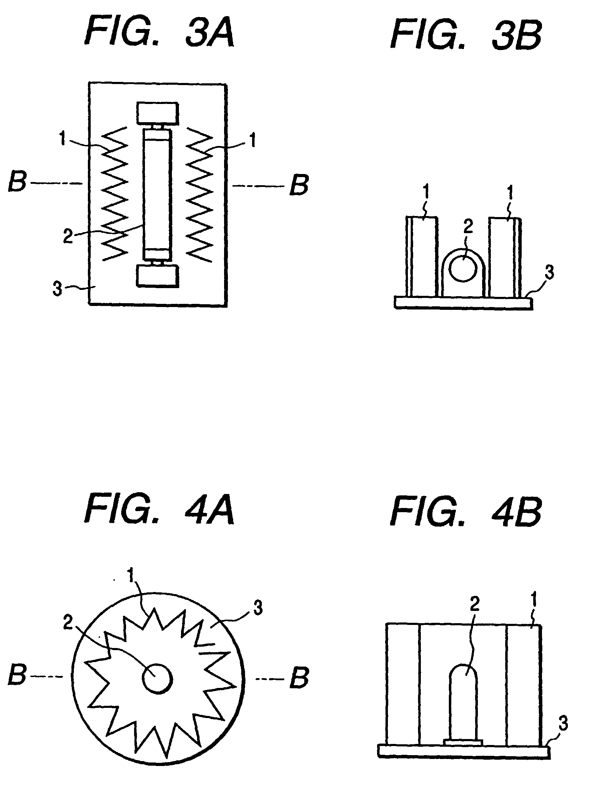

Fig. 3A illustrates an example of the air cleaning unit of the invention, and Fig. 3B is a cross section of Fig. 3A along line B-B. The air cleaning unit shown has a UV-generator 2 that is fixed on a mount 3 horizontally and a photocatalyst sheet 1 that is set upright on each side of the UV-generator 2. The UV-generator 2 includes those generating UV light of 400 nm or shorter wavelengths, such as a blacklight blue lamp, a fluorescent lamp, a halogen lamp, a xenon flash lamp, a mercury lamp, and a germicidal lamp. The photocatalyst sheet 1 is folded (pleated) to provide an increased surface area. An increased surface area can also be obtained by using a honeycomb structure, etc. made of the photocatalyst sheet instead of the folded photocatalyst sheet 1.

The photocatalyst sheet 1 is set in the vicinities of a UV-generator so that it can be efficiently irradiated with ultraviolet rays emitted from the UV-generator. For example, as shown in Fig. 4A (plan view) and Fig. 4B (cross section of Fig. 4A along line B-B), a UV lamp 2 is put upright on a mount 3, and a pleated photocatalyst sheet 1 is set upright on the mount 3 to surround the UV lamp 2. If necessary, a reflective mirror can be set.

Since air diffuses and passes through the voids between the photocatalyst particles and the resin binder, air comes into contact with the wide surface area of the photocatalyst particles so that deodorizable components in air can be oxidized and deodorized with the activated photocatalyst particles. Being hardly decomposable, the polytetrafluoroethylene that is a binder resin supporting the photocatalyst particles can retain its shape while enveloping the photocatalyst particles stably for a long time. Therefore, the sheet is capable of oxidatively decomposing deodorizable components in air for a prolonged period of time.

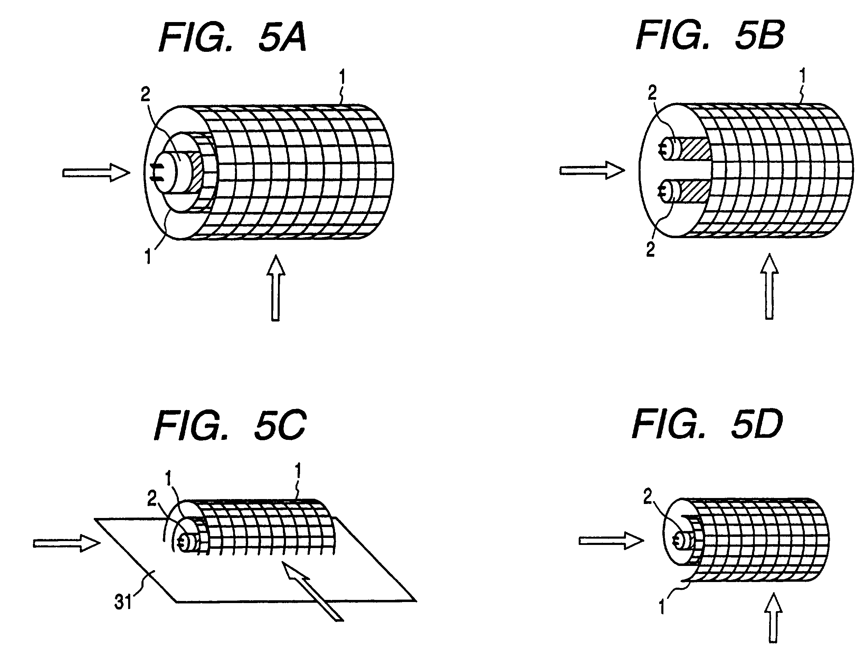

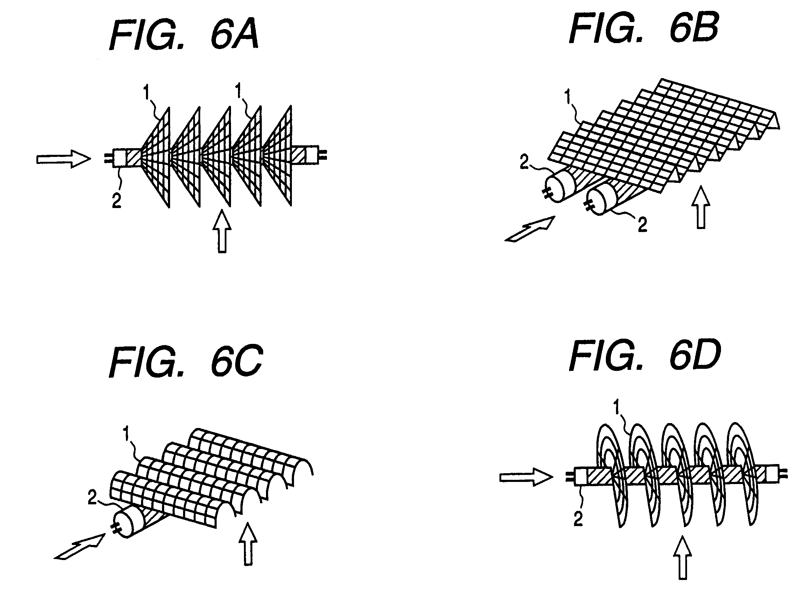

Figs. 5 and 6 show a side or perspective view of other examples of the air cleaning unit of the invention, in which an air-permeable photocatalyst sheet 1, which is set within the area receptive of light from an ultraviolet generator 2, is shaped into a circular cylinder, an elliptic cylinder, an arc, a whirlpool (loose roll), an umbrella, pleats, waves, a spiral, etc. and put into a case having a filter and a fan at the air inlet and outlet, respectively. The arrows in these figures indicate the flow of air on which the air cleaning unit is effective.

More specifically, the unit shown in Fig. 5A has two concentric cylinders formed of an air-permeable photocatalyst sheet 1 containing a UV lamp 2 in their center so that each sheet is uniformly irradiated with UV light at a fixed distance. This structure is suitable for a compact air cleaning unit. By surrounding the outer cylinder with a cylindrical reflective mirror made of, e.g., an aluminum plate, the outer side of each sheet can be made use of for air cleaning. The structure is effective for both an air flow parallel to the UV lamp and an air flow perpendicular to the lamp.

The unit shown in Fig. 5B has an air-permeable photocatalyst sheet 1 shaped into an elliptic cylinder containing a U-shaped UV lamp or two parallel UV lamps, the focuses of the ellipse agreeing with the axes of the lamp(s). This structure is practical for a compact air cleaning unit. The outer side of the sheet 1 can be made use of for air cleaning by surrounding the cylinder by a cylindrical reflective mirror made of, e.g., an aluminum plate. The structure is effective on a parallel and a perpendicular air flow to the UV lamp. The U-shaped UV lamp, being fixed at one end of the cylindrical sheet, is advantageous for exchange and cleaning of the photocatalyst sheet.

The unit shown in Fig. 5C comprises a reflective plate 31, a UV lamp 2 fitted on the reflective plate 31, and two air-permeable photocatalyst sheets 1 arched over the UV lamp 2 at different curvatures.

The unit shown in Fig. 5D has an air-permeable photocatalyst sheet 1 rolled up loosely around a UV lamp 2. According to this structure, practically the same performance as is exhibited by the structure shown in Fig. 5A can be obtained by using a single sheet.

The unit shown in Fig. 6A comprises a plurality of air-permeable photocatalyst sheets 1 each shaped into an umbrella and fitted around the UV lamp 2. An air flow parallel to the lamp can be cleaned through multiple stages.

The unit shown in Fig. 6B has an air-permeable photocatalyst sheet 1 folded into pleats and set on an UV lamp 2 to make a planar structure, which can be used in combination with sunlight. A reflective plate may be provided in the same manner as in Fig. 5C.

The unit shown in Fig. 6C has a corrugated air-permeable photocatalyst sheet 1 set on a UV lamp 2. Being planar, the structure can be used in combination with sunlight similarly to the unit of Fig. 6B. A reflective plate may be provided in the same manner as in Fig. 5C.

The unit shown in Fig. 6D has an air-permeable photocatalyst sheet 1 shaped into spiral stairs having a UV lamp 2 in the axis of the spiral. An air flow parallel to the lamp can be cleaned through multiple stages.

The air cleaning unit according to the present invention is suited for removal or reduction of bad odors such as the odor of tobacco, harmful gases such as $NO_x$ and $SO_x$, volatile organic compounds (generated from new houses) and microorganisms in a living space or a working environment.

The air cleaning unit of the invention achieves high efficiency in air cleaning because of the continuous void structure of the photocatalyst layer. In particular, the units shown in Figs. 5 and 6 having an appropriately shaped air-permeable photocatalyst sheet offer the following advantages. (1) The air-permeable photocatalyst sheet is shaped to provide a structure in which air is brought into contact with the photocatalyst many times while passing through the unit. (2) Air is agitated while passing through the mesh of the air-permeable photocatalyst sheet. (3) Where a reflective mirror is provided, the back side of the air-permeable photocatalyst sheet opposite to the side facing an ultraviolet generator is activated by the reflected light so that the air having passed through the sheet is again treated by the back side of the sheet. (4) Since the structure copes with either of two air flow directions, it has freedom of design for setting an ultraviolet generator to achieve high efficiency in air cleaning. The freedom of design combined with the simplicity of shaping the photocatalyst sheet provides a compact and inexpensive air cleaning means.

The present invention will now be illustrated in greater detail with reference to Examples, but it should be understood that the invention is not limited thereto.

EXAMPLE 1

A sheet of 60 μm thick aluminum foil was dip coated with an aqueous dispersion of polytetrafluoroethylene powder (particle size: 0.3 μm; specific gravity: 2.20) containing 40 wt% of titanium oxide particles (particle size: 7 nm; specific gravity: 3.84), dried at 100°C, and baked at 390°C for 2 minutes to obtain a photocatalyst sheet comprising a photocatalyst layer having a thickness of 7 μm and a void volume of 12.2%.

A 10 cm wide and 100 cm long piece was cut out of the photocatalyst sheet and pleated to have an apparent size of 10 cm x 10 cm. The pleated piece was set on each side of a black blue light (4W; tube length: 11.5 cm) at a distance of 3 cm from the light to prepare an air cleaning unit.

The resulting air cleaning unit was placed in the toilet room (floor space: about 0.9 m x 1.8 m) of 8 ordinary Japanese houses where bad odor was always perceived. The bad odor was removed in 6 toilet rooms after one day from the placement and in 2 toilet rooms after two days from the placement.

EXAMPLE 2

A photocatalyst sheet was prepared in the same manner as in Example 1, except for replacing the aluminum foil with carbon fiber felt having a thickness of 0.6 mm and a basis weight of 30 g/m$^2$. A commercially available deodorant sheet mainly comprising copper carboxymethylcellulose (Clean Sky, produced by Kohjin Co., Ltd.) was laminated on the photocatalyst sheet. A 10 cm wide and 100 cm long piece was cut out of the laminated sheet and pleated to have an apparent size of 10 cm x 10 cm. A hook was attached to each end of the pleated piece. An air cleaning unit was assembled using the pleated piece in the same manner as in Example 1 and placed in a chemicals storage (floor space: about 0.9 m x 1.8 m) which always had an unpleasant odor. The unpleasant odor was gotten rid of after 5 hours from the placement.

COMPARATIVE EXAMPLE 1

An air cleaning unit was prepared in the same manner as in Example 1, except for replacing polytetrafluoroethylene with a perfluoroalkyl vinyl ether-tetrafluoroethylene copolymer having a melt viscosity of

$10^4$ poise. The void volume of the photocatalyst layer was 1%. The air cleaning unit was tested in toilet rooms in the same manner as in Example 1 but failed to carry out satisfactory deodorizing.

EXAMPLE 3

An air cleaning unit was prepared in the same manner as in Example 1, except that the photocatalyst layer of the photocatalyst sheet had a void volume of 3.1% and a thickness of 7 µm. When the unit was tested in the same manner as in Example 1, the deodorizing efficiency achieved was lower than that of Example 1 but more satisfactory than in Comparative Example 1.

The inferiority in deodorizing performance of the air cleaning unit of Comparative Example 1 to those of Examples 1 to 3 is assumed to be because the perfluoroalkyl vinyl ether-tetrafluoroethylene copolymer is fused to the photocatalyst particles on baking to cover most of the surface area of the photocatalyst particles and also because the copolymer hardly retains its powdered form while being baked due to its low melt viscosity, leaving little vacancy among sintered particles.

EXAMPLE 4

Glass fiber cloth of plain weave (fiber thickness: 0.5 mm; mesh size: 2.5 mm x 2.5 mm; porosity: about 30%) was dip coated with the same dispersion as used in Example 1, dried at 110°C for 60 seconds to remove the water content, and baked at 370°C for 100 seconds to obtain an air-permeable photocatalyst sheet having a thickness of 0.55 mm. The amount of titanium oxide particles adhered to the cloth was 90 g/m$^2$.

COMPARATIVE EXAMPLE 2

An air-permeable photocatalyst sheet was prepared in the same manner as in Example 4, except for replacing polytetrafluoroethylene of the dispersion with a perfluoroalkyl vinyl ether-tetrafluoroethylene copolymer.

A 200 mm wide and 300 mm long piece was cut out of each of the air-permeable photocatalyst sheets obtained in Example 4 and Comparative Example 2. A blacklight (10 W) was put in a 2 m$^3$-volume closed container, and the cut piece of the sheet was curved into an arch and set over the blacklight at a distance of about 10 cm to make an air cleaning unit. The container was filled with acetaldehyde in a concentration of 10 ppm, and the blacklight was turned on. The UV intensity on the sheet was 1 mW/cm$^2$. After 180 minutes, the aldehyde concentration in the container was measured by gas chromatography. As a result, the concentration in the container having the air cleaning unit of Example 4 was found reduced to 3 ppm, whereas that in the container having the unit of Comparative Example 2 was as high as about 3 times that of Example 4.

While the invention has been described in detail and with reference to specific examples thereof, it will be apparent to one skilled in the art that various changes and modifications can be made therein without departing from the spirit and scope thereof.

**Claims**

1. An air cleaning unit having a photocatalyst sheet comprising a supporting substrate having thereon a photocatalyst layer comprising a baked polytetrafluoroethylene resin having dispersed therein photocatalyst particles with fine voids formed between said resin and said photocatalyst particles, said photocatalyst sheet being placed in an area receptive of light from an ultraviolet generator.

2. The air cleaning unit according to claim 1, wherein said photocatalyst sheet has laminated thereon a deodorant sheet mainly comprising one or more of activated carbon, zeolite and copper carboxymethylcellulose.

3. The air cleaning unit according to claim 1, wherein said photocatalyst sheet is a curved or folded air-permeable sheet comprising a mesh or perforated supporting substrate having thereon said photocatalyst layer.

4. The air cleaning unit according to claim 1, wherein said photocatalyst layer has a void volume of 7% or more.

5. The air cleaning unit according to claim 1, wherein the photocatalyst layer contains the photocatalyst particles in an amount of 5 to 60 % by weight.

6. The air cleaning unit according to claim 1, wherein the photocatalyst layer has a thickness of from 3 to 30 µm.

7. The air cleaning unit according to claim 1, wherein the photocatalyst particles are selected from the group consisting of includes titanium oxide, strontium titanate, tungsten oxide, zinc oxide, tin oxide, and cadmium sulfide.

8. The air cleaning unit according to claim 1, wherein the photocatalyst particles are anatase type titanium oxide particles.

# FIG. 1

# FIG. 2A

# FIG. 2B

*FIG. 3A*

*FIG. 3B*

*FIG. 4A*

*FIG. 4B*

FIG. 5A

FIG. 5B

FIG. 5C

FIG. 5D

EP 0 978 690 A2

# FIG. 6A

# FIG. 6B

# FIG. 6C

# FIG. 6D

EP 0 978 690 A2